(19) **Europäisches Patentamt**
**European Patent Office**
**Office européen des brevets**

(11) **EP 1 920 784 A1**

(12) **EUROPEAN PATENT APPLICATION**

(43) Date of publication:
**14.05.2008 Bulletin 2008/20**

(51) Int Cl.:
*A61K 41/00* (2006.01)

(21) Application number: **06123943.0**

(22) Date of filing: **13.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicants:
- **Koninklijke Philips Electronics N.V.**
**5621 BA Eindhoven (NL)**
Designated Contracting States:
**FR GB**
- **Philips Intellectual Property & Standards GmbH**
**20099 Hamburg (DE)**
Designated Contracting States:
**DE**

(72) Inventor: **The designation of the inventor has not yet been filed**

(74) Representative: **Van Velzen, Maaike Mathilde**
**Philips**
**Intellectual Property & Standards**
**P.O. Box 220**
**5600 AE Eindhoven (NL)**

(54) **Radiation sensitizers in ionizing radiation therapy and imaging**

(57) The invention relates to enhancing the effects of ionizing radiation (8), by providing nanoparticles (6) having material composition with a higher efficiency in producing solvated electrons or $O_2^-$ or $OH^-$ radicals having a malicious effect in tissue. The nanoparticles (6) comprise one or more non-metallic reducing agents compounded to display electronic band structure. In one embodiment, the nanoparticles may comprise redox-active oxidic material such as $TiO_2$ compounded to display electronic band structure.

Fig. 1

**Description**

FIELD OF THE INVENTION

The present invention relates to radiation sensitizer for enhancing the effect of electromagnetic radiation therapy, such as for use in cancer treatment.

BACKGROUND OF THE INVENTION

The use of electromagnetic radiation in cancer treatment is based on cytotoxic substances, radicals or ions, formed by the radiation-matter interaction in the tissues. The applied radiation can be divided into:

- Exciting radiation forming exited molecular or atomic states such as radicals. Here, lower energy radiation, typically IR, visible and near-UV light, is used. This application is commonly referred to as *photodynamic therapy* or PDT.
- Ionizing radiation forming ions and free electrons. This requires high-energy radiation, typically far-UV (FUV), extreme-UV (EUV), X-rays and gamma rays. This application is commonly referred to as *radiation therapy.*

The distinction is considerable due to the very different processes. It should be mentioned that low energy radiation can be ionizing through multi-photon absorption processes at extremely high intensities. Such intensities, however, bums or vaporize materials under normal circumstances and are therefore not relevant for generating cytotoxic substances in tissue.

In PDT, the excitation of a photosensitizer molecule in the presence of oxygen ($O_2$) generates reactive oxygen species, such as singlet oxygen ($^1O_2$) which is a serious cytotoxic agent, see e.g. Wang et al., J. Mater. Chem. 14 (2004), 487-493.

In radiation therapy, the high-energy photons interact with molecules in the tissue (especially water), causing the ejection of high-energy electrons, i.e. ionization. These electrons can exert a cytotoxic effect directly by, e.g. breaking the sugar phosphate bonds in DNA. More frequently, however, they exert their effect indirectly via the follow-up formation of solvated electrons and hydroxyl radicals:

$$H_2O \; \xrightarrow{\text{X-rays}} \; H_2O^+ + \; e^-_{aq} \; \xrightarrow{H_2O} \; H_3O^+ + HO^\cdot + e^-_{aq}$$

Hydroxyl radicals are highly reactive, oxidizing entities that cause damage within about 4nm three-dimensional radius of where they are formed.

The hydrated electrons however, hydrated electrons are solvated electrons in watery solution, are strongly reducing, rather mobile, and relatively long-lived ($T_{1/2} \sim 25$ ns). Although the hydrated electrons can cause DNA damage themselves, they are also capable of recombining with hydroxyl radicals:

$$e^-_{aq} + HO^\cdot \; \rightarrow \; HO^- ,$$

which effectively deactivates both cytotoxic products, the hydrated electron and the hydroxyl radical.

In the presence of oxygen, the hydrated electrons get trapped, at least initially, in the form of superoxide anion.

$$e^-_{aq} + O_2 \; \rightarrow \; O_2^{\cdot -}$$

This trapping consumes the hydrated electrons and thereby reduces the deactivation of the hydroxyl radicals, leaving the latter free to carry out cell killing. Hence, high oxygen content will improve the cytotoxic effect indirectly. A major difference between many solid tumors and surrounding normal tissue is the presence in tumors of chronically hypoxic, yet still viable cells. Hypoxic cells in solid tumors contain a lower oxygen tension (and correspondingly reduced number of $e^-_{aq}$ traps) than their highly vascularized, rapidly growing "outside neighbors". This rationalizes why hypoxic cells have been observed to be 2.5-3.0 times more resistance to the damaging effects of ionizing radiation. While cure or control rates of a tumor can be elevated with an effective increase in radiation dose, such increase also tends to damage adjacent, fully-oxygenated tissues as well as the targeted neoplastic ones. Consequently, failure of conventional radiation therapy and local recurrences are common events that are in large measure attributed to the presence of radiation-resistant hypoxic cell populations in solid tumors. Specific modification of tumor radiation sensitivity has been pursued through alteration of the tumor oxygenation state. This can be achieved through the use of fractionation, wherein the total radiation dose is divided into multiple smaller doses. This protocol allows healthy tissue time to undergo cell repopulation and permits reoxygenation of hypoxic regions originally distant from functional vasculature. Unfortunately, even this approach, does not provide a complete, satisfactory solution to the problems of hypoxic-region cancer control.

Specific modification of tumor radiation sensitivity can be achieved, at least in principle by the use of so-called radiation sensitizers. Radiation sensitizers are pharmacologic agents that increase the lethal effects of radiation when administered in concert with it. During the past 30 years, several chemical agents and drug products approved for other indications have been studied as possible radiosensitizers.

US 2005/0020869 discloses gold nanoparticles to deliver a very large quantity of gold to tumors via intravenous injection without apparent toxicity and showed that the combination with X-rays resulted in eradication of most tumors. It is a disadvantage that gold colloids are prone to coagulation, resulting in much larger particles. For this reason, the colloidal solutions have to be either prepared freshly, which is not easy in a hospital environment, or they have to be stabilized.

## SUMMARY OF THE INVENTION

Accordingly, the invention preferably seeks to mitigate, alleviate or eliminate one or more of the above mentioned disadvantages singly or in any combination. In particular, it may be seen as an object of the present invention to provide radiation sensitizers that solve the above mentioned problems by further increasing the number of hydrated electrons generated upon exposure to ionizing electromagnetic radiation.

In general, the physical and chemical processes relevant within the context of the present invention occurs in, or in the vicinity of, animal or plant cells and thus in an at least substantially liquid environment.

This object and several other objects are obtained in a first aspect of the invention by providing nanoparticles comprising non-metallic reducing agents compounded to display electronic band structure for use as radiation sensitizers.

In a preferred embodiment, the nanoparticles according to the second and third aspects may be for use as radiation sensitizers in radiation therapy applying ionizing electromagnetic radiation. In another preferred embodiment, the nanoparticles according to the second and third aspects may be for use as radiation sensitizers in diagnostic imaging methods applying ionizing electromagnetic radiation.

The invention is particularly, but not exclusively, advantageous for obtaining an increase in the number of generated hydrated electrons. This increase is brought about by the oxidation of the compounded non-metallic reducing agents of the nanoparticles in addition to the oxidation of the naturally occurring agents, primarily water.

In the present context, a non-metallic reducing agent is a substance that can be easily oxidized and thereby act as a donor of solvated electrons or $O_2^-$ or $OH^-$ radicals, e.g. a redox-active oxidic material such as $TiO_2$.

That the substance is compounded to display electronic band structure means that the atoms/molecules/ions are congregated in a structure with the property of electronic band structure. In case of electronic band structures, valence electrons of individual atoms have become delocalized. In principle only infinitely large single crystals have an electronic band structure, but systems approaching infinity also have a well developed electronic band structure. Nanocrystals are sufficiently large to develop a band like electronic structure, albeit the band structure is size dependent. Although electronic band structures are usually associated with crystalline materials, quasi-crystalline and amorphous solids such as molecular clusters can exhibit band structures in the sense that electrons and holes are delocalized in the complete particle.

By ionizing electromagnetic (EM) radiation is meant photons capable of producing hydrated electron instead of only high-energy excited states by single-photon absorption processes. Thus, it is understood that the exact threshold energy depends on the band gap of the compound reducing agent. In an alternative definition, ionizing EM radiation is FUV radiation, EUV radiation, X-rays, gamma-rays or EM radiation with a wavelength $\lambda < 200nm$ or a photon energy $E_{hv} > 6,2eV$.

A nanoparticle is a particle having at least one dimension (e.g. largest diameter/diagonal) smaller than 200nm. In a preferred embodiment of the invention, a nanoparticle is a particle having a dimension in the range 2nm - 100nm.

In a second aspect, the invention provides the use of non-metallic reducing agents compounded to display electronic band structure for the manufacture of nanoparticle radiation sensitizers for radiation therapy and/or diagnostic imaging applying ionizing electromagnetic radiation.

And in a similar, third aspect, the invention provides the use of nanoparticles composed entirely by non-metallic reducing agents compounded to display electronic band structure for the manufacture of radiation sensitizers for radiation therapy and/or diagnostic imaging applying ionizing electromagnetic radiation.

In a fourth aspect, the invention provides a method of enhancing the effects of ionizing radiation directed to a substance comprising administering an amount of nanoparticles to a substance in vitro and subsequently irradiating the substance in vitro with ionizing electromagnetic radiation, wherein said nanoparticles are characterized in that they have at least an outer surface layer composed of one or more non-metallic reducing agents compounded to display electronic band structure. The enhancing of the effect is preferably brought about by an increased generation of hydrated electrons by the compound reducing agent.

Similarly, in a fifth aspect, the invention provides a method of enhancing the effects of radiation directed to a tissue or a population of cells in an animal comprising administering an amount of nanoparticles to at least said tissue or population of cells and subsequently irradiating the animal with ionizing electromagnetic radiation directed to said tissue or population of cells, wherein said nanoparticles are characterized in that they have at least an outer surface layer composed of one or more non-metallic reducing agents compounded to display electronic band structure. The method according to the fifth aspect may be limited to non-therapeutic and non-diagnostic applications.

It may be preferred that the nanoparticles are composed entirely by non-metallic reducing agents compounded to display electronic band structure. However, in all but the third aspect, nanoparticles may preferably be composed by an inner metal core and an outer surface layer consisting of the compounded non-metallic reducing agents. This structure provides the advantage that the metal core can be multiply ionized (even in both directions: oxidation and reduction) relatively easily as the electric charges can be delocalized over the complete metallic core. In this way, the core can provide quite a few electric charges to the non-metallic reducing agents, in this way the non-metallic reducing agents can be used several times. For the core to be metallic it does not need to be very large (20-30 atoms might be sufficient), however the process of charge delocalization is more effective for larger cores.

The embodiments where the nanoparticles are composed entirely by the compounded non-metallic reducing agents have the advantage of simple fabrication and large possible variety in size. In addition, the generated charges are long-lived, as they can be trapped at e.g. lattice defect sites. This trapping is favored by initial delocalization of the charges involving the band structure of the particles, followed by localization. Especially the surface of materials is highly defective. This is beneficial as the charge transfer required profits from a close proximity. It is another advantage that such particles might be made very specific with respect to excitation with visible light, by choosing the right chemical composition.

In the first through fifth and other aspects, the compounded non-metallic reducing agents may be characterized further. In a first preferred embodiment, the applied non-metallic reducing agents may comprise one or more oxidic reducing agents, such as oxides selected from the group consisting of: $TiO_2$, $ZrO_2$, $HfO_2$, $V_2O_5$, $Nb_2O_5$, $Ta_2O_5$, $MoO_3$, $WO_3$, $Ga_2O_3$, $In_2O_3$, $SnO_2$, $CeO_2$, $Pr_6O_{11}$, $Nd_2O_3$, $Tb_4O_7$, $Dy_2O_3$, and $Eu_2O_3$. The oxides are preferably compound in large molecular clusters to form particles governed by solid state properties.

In another preferred embodiment, the applied non-metallic reducing agents may comprise semiconductor materials doped with metal cations such as $Ce^{3+}$, $Pr^{3+}$, $Nd^{3+}$, $Eu^{3+}$, or $Yb^{2+}$. Here, nanoparticles may be formed as doped semiconductor nanoparticles displaying electronic band structure (also referred to as quantum dots).

The above preferred embodiments for the non-metallic reducing agents has the following advantages:

- They stabilize the resulting nanoparticles against coagulation which simultaneously adding the functionality of reducing agent.
- They provide charge localization; dielectric particles generally contain defects, such as anion vacancies. Such defects can be charged easily, localizing the charge but with a relatively high energy. In the close proximity of water or oxygen, this charge will be transferred these moieties, thus adjusting the desired effect. Similar charge localization is provided by semiconductor particles (quantum dots) doped metal ions with more than one valence state. This is not possible in metallic particles, as charge cannot be localized in electrically conductive materials.

In particular, the band structure of the nanoparticles provides the following advantages:

- It allows adjustable but broad absorption spectrum (by changing the material)
- The excited state relaxation (depends on the material) allows localization of the charge carriers which prevents a too fast recombination.
- Systems with a band structure can easily be doped with donors/acceptors. In this way additional oxidation/reduction power is achieved.

These may be compared to the disadvantages of small molecules, such as those often used for PDT. Here, the electrons and holes are necessarily in each others vicinity, this favors fast direct recombination which reduces their effect. Also, small molecules do not survive prolonged excitation with ionizing radiation, in contrast to larger units

The basic idea of the invention relates to enhancing the effects of ionizing radiation, by providing nanoparticles comprising one or more non-metallic reducing agents compounded to display electronic band structure, which have a higher efficiency in producing solvated electrons or $O_2^-$ or $OH^-$ radicals.

The first through fifth and other aspects of the invention will be apparent from and elucidated with reference to the embodiments described hereinafter.

BRIEF DESCRIPTION OF THE DRAWINGS

The present invention will now be explained, by way of example only, with reference to the accompanying Figures, where

Figures 1A-E are illustrations showing an embodiment of the use of radiation sensitizers according to the invention.
Figure 2 illustrates the detailed physical process of generation of hydrated electrons.
Figure 3 illustrates a nanoparticle with a core-shell structure according to an embodiment of the invention.

DETAILED DESCRIPTION OF PREFERRED EMBODIMENTS

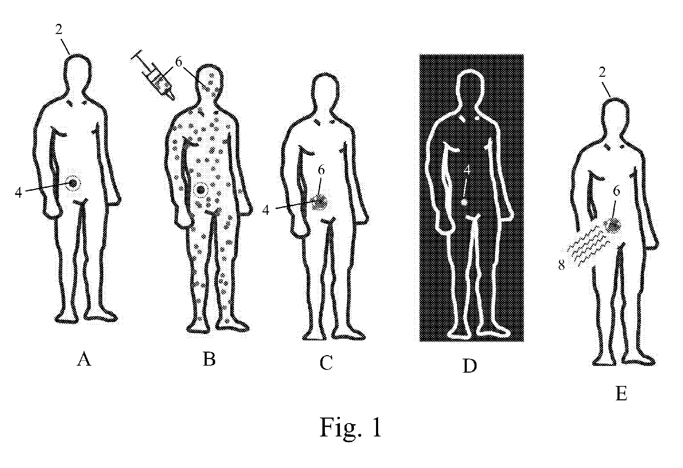

Figure 1A-E illustrates an embodiment of the uses according to the invention. Here (1A), a patient 2 has a malicious tissue portion 4. Radiation sensitizers in the form of nanoparticles 6 formed by $TiO_2$ compounded to display electronic band structure are injected (1B) into the patient. Due to targeting bioligands attached thereto, the nanoparticles congregate (1C) at the malicious tissue portion 4.

The nanoparticles may be, or may be functionalized to be, traceable so that the exact location of the malicious tissue portion 4 can be determined by determining congregation of nanoparticles in a scanning step (1D). Nanoparticles with metal core can be used as contrast agent in ultrasound imaging, Gadolinium functionalized nanoparticles can be used as contrast agent in MRI, and Technetium functionalized ($^{99m}$Tc) nanoparticles can be used as contrast agent in SPECT (Single photon emission computed tomography).

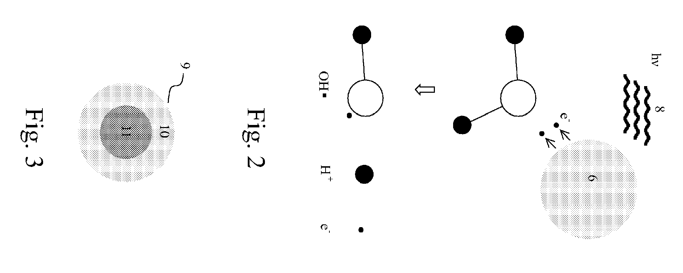

Once the malicious tissue portion 4 is localized and populated with of nanoparticles 6, the radiation therapy can start (1E). A detailed illustration of the mechanism of the radiation therapy is shown in Figure 2. Ionizing radiation 8 incident on the nanoparticles 6 results in a much larger production of hydrated electrons than in surrounding tissue.

The hydrated electrons can exert a cytotoxic effect directly and can react with water (water being the most abundant substance in the surroundings) to produce highly cytotoxic hydroxyl radicals and hydrated electrons:

$$2H_2O + e^-_{aq} \rightarrow H_3O^+ + HO^. + 2\,e^-_{aq}$$

Hence, high-energy radiation will be converted into smaller energy packages by the materials of the present invention, and this energy is used to produce the solvated electrons.

In a specific embodiment of the use described in relation to Figures 1A-E and Figure 2, $TiO_2$ nanoparticles (diameter ~ 5nm) are injected into the cancerous tissue. Upon irradiation with UV radiation (hv < 400 nm), $TiO_2$ cleaves water molecules into protons, electrons and OH radicals after band gap excitation.

Fabrication

Nanoparticles of reducing agents consisting of rare-earth (RE) oxides can be prepared by dissolving e.g. the corresponding nitrates in doubly distilled and microfiltrated water in amounts yielding several grams of the RE oxides per liter. After having dissolved the rare-earth nitrate, the pH value is slowly adjusted to about 4.0 at room temperature, using nitric acid. Urea is added e.g. in a 40-fold excess. By heating this solution, the pH-value increases and at pH values of about 5.5- 6.0 a precipitate is formed. This precipitate consists of $RE(OH)CO_3$. By careful heating at temperatures above about 635 °C, the oxide is formed, which consists of very small particles. A milling step can be included to reduce agglomeration. Particle control is best for small RE concentrations (f.i. resulting in 1 g / 1 $H_2O$),

Alternatively, spray drying can be used. In this method, a precursor of the material to be obtained in nanocrystalline form is dissolved in preferably an uninflammable liquid. In a spray dryer, this solution is fed drop-wise into a hot nozzle. The solvent very quickly evaporates and the resulting nanoparticles are quickly transported away by an air stream and collected. These materials can be treated thermally mildly afterwards to improve e.g. their crystalline properties and be milled to reduce agglomeration.

Nanoparticles of reducing agents consisting of semiconductor material doped with metal cations can be fabricated by doping appropriate quantum dots (quantum dots being semiconductor nanoparticles displaying band structure). A number of well known fabrication techniques for quantum dots exist, such as colloidal synthesis, molecular beam epitaxy (MBE), metallorganic vapor phase epitaxy (MOVPE), and electron beam lithography.

It is possible to fabricate the nanoparticles with a core -shell structure as illustrated in Figure 3. In this case, a nanoparticle can consists of a metal or semiconductor core material 11, overcoated with a shell material 10 of reducing agent compounded to display electronic band structure. As an example, consider $Y_2O_3$ nanoparticles fabricated by the routes described in the example given above (Urea). The nanoparticles obtained, in turn can be coated with $Eu_2O_3$. $Eu_2O_3$ is precipitated on $Y_2O_3$ in the same manner as the preparation of the $Y_2O_3$ nanoparticles, albeit that after micro filtrating the water, the pH value is adjusted to 4.5 -5.0 and that $Y_2O_3$ is added thereafter. Alternatively, a soluble Eu-salt, e.g. the nitrate, is added to an $Y_2O_3$ suspension and this suspension is spraydried. This method can also be used to coat metallic nanoparticles.

Targeting

The radiation sensitizer nanoparticles according to the invention can be made to target specific tissues by incorporating in the outer material layers, or by linking or attaching the particle to, a molecule that has affinity for the target tissue. Such molecule is also referred to as a "targeting molecule", or a "targeting moiety" or a "targeting ligand", which can be an antibody, antibody fragment, single chain antibody, peptide, nucleic acid, carbohydrate, lipid, lectins, drug, or any other compound that binds to the target tissue.
Typical targeting units may be:

- Antibodies (monoclonal, polyclonal, mouse, mouse-human chimeric, human, single-chain, diabodies, etc), like Trastuzumab (breast cancer), Rituximab (non-Hodgkin-lymphoma), Alemtuzumab (chronial-lymphozytic leukemia); Gemtuzumab (acute myelogene leukemia); Edrecolomab (colon cancer); Ibritumomab (non-Hodgkin-lymphoma); Cetuximab (colon cancer); Tositumomab (non-Hodgkin-lymphoma); Epratuzumab (non-Hodgkin-lymphoma); Bevacizumab (lung and colon cancer); anti-CD33 (acute myelogene leukemia); Pemtumomab (ovarian and stomach cancer); Mittumomab (lung and skin cancer); anti-MUC 1 (adenocarcinoma); anti-CEA (adenocarcinoma); anti-CD 64 (plaques), etc.
- Peptides, Polypeptides, Peptidomimetics, like Somatostatin analogs, vasoactive peptide analogs, neuropeptide Y, RGD peptides, etc.
- Proteins, like Annexin V, tissue plasminogen activator protein, transporter proteins, etc.
- Macromolecules, e.g., Hyaluronan, Apcitide, Dermatan sulfate
- Nucleic acids, like Apatamers, anti-sense DNA/RNA,/PNA, small-interfering RNAs, etc.
- Lipids, like Phospholipids, etc.
- Lectins, e.g. Leukocyte stimulatory lectin
- Saccharides
- Small molecules

Nanoparticles containing a targeting molecule can be made in several ways. For example, a targeting molecule can be incorporated in a nanoparticle during the synthesis of the nanoparticle. Alternatively, the surface of the nanoparticles can be modified after synthesis.
The nanoparticles can be functionalized by the spray drying technique, i.e. the surface of the nanoparticles reacts with ligands during spray drying. Alternatively, complex molecules like EDTA can be used to bind on both the nano-particles and the ligands, e.g. by a reaction in $H_2O$.
In order to modify the surface of the doped quantum dots with transfer molecules, they can be subjected to standard capping exchange procedure. An excess amount of transfer molecules with suitable functional groups are added to the QD solution in chloroform and stirred at 50 °C for several hours. Methanol can be used to precipitate the QDs. The dissolution and precipitation steps are repeated several times in order to remove transfer molecules which were not bound to QD surfaces. The transfer molecules can be chosen to have a small distance to the QD by introduction of a functional group which attaches directly to the surface of the QD, e.g., through a carboxylate, phosphonate, sulphonate or thiol-group.

**Claims**

1. Nanoparticles (6, 9) comprising non-metallic reducing agents compounded to display electronic band structure for use as radiation sensitizers.

2. The use according to claim 1, wherein the nanoparticles (6) are composed entirely by non-metallic reducing agents compounded to display electronic band structure.

3. The use according to any of the preceding claims, wherein the nanoparticles are used as radiation sensitizers in radiation therapy applying ionizing electromagnetic radiation.

4. The use according to any of the preceding claims, wherein the nanoparticles are used as radiation sensitizers in diagnostic imaging methods applying ionizing electromagnetic radiation.

5. The use according to any of the preceding claims, wherein the non-metallic reducing agents comprise one or more oxidic materials selected from the group consisting of:

$TiO_2$, $ZrO_2$, $HfO_2$, $V_2O_5$, $Nb_2O_5$, $Ta_2O_5$, $MoO_3$, $WO_3$, $Ga_2O_3$, $In_2O_3$, $SnO_2$, $CeO_2$, $Pr_6O_{11}$, $Nd_2O_3$, $Tb_4O_7$, $Dy_2O_3$, and $Eu_2O_3$.

6. The use according to any of claims 1-4, wherein the non-metallic reducing agents comprise semiconducting materials doped with metal cations selected from the group consisting of: $Ce^{3+}$, $Pr^{3+}$, $Nd^{3+}$, $Eu^{3+}$, or $Yb^{2+}$.

7. Use of non-metallic reducing agents compounded to display electronic band structure for the manufacture of nanoparticle radiation sensitizers for radiation therapy and/or diagnostic imaging applying ionizing electromagnetic radiation.

8. The use according to claim 7, wherein the manufactured nanoparticles (9) are composed by an inner metal core (11) and an outer surface layer (10) consisting of the compounded non-metallic reducing agents.

9. Use of nanoparticles (6, 9) composed entirely by non-metallic reducing agents compounded to display electronic band structure for the manufacture of radiation sensitizers for radiation therapy and/or diagnostic imaging applying ionizing electromagnetic radiation.

10. The use according to any of claims 7-9, wherein the non-metallic reducing agents comprise one or more oxidic materials selected from the group consisting of: $TiO_2$, $ZrO_2$, $HfO_2$, $V_2O_5$, $Nb_2O_5$, $Ta_2O_5$, $MoO_3$, $WO_3$, $Ga_2O_3$, $In_2O_3$, $SnO_2$, $CeO_2$, $Pr_6O_{11}$, $Nd_2O_3$, $Tb_4O_7$, $Dy_2O_3$, and $Eu_2O_3$.

11. The use according to any of claims 7-10, wherein the non-metallic reducing agents comprise semiconducting materials doped with metal cations selected from the group consisting of: $Ce^{3+}$, $Pr^{3+}$, $Nd^{3+}$, $Eu^{3+}$, or $Yb^{2+}$.

12. A method of enhancing the effects of ionizing radiation directed to a substance comprising administering an amount of nanoparticles (6, 9) to a substance in vitro and subsequently irradiating the substance in vitro with ionizing electromagnetic radiation,
wherein said nanoparticles are **characterized in that** they have at least an outer surface layer composed of one or more non-metallic reducing agents compounded to display electronic band structure.

13. A method of enhancing the effects of radiation directed to a tissue or a population of cells in a human or an animal comprising administering an amount of nanoparticles (6, 9) to at least said tissue or population of cells and subsequently irradiating the animal with ionizing electromagnetic radiation directed to said tissue or population of cells,
wherein said nanoparticles are **characterized in that** they have at least an outer surface layer composed of one or more non-metallic reducing agents compounded to display electronic band structure.

14. The method according to claim 13, wherein the method is non-therapeutic and non-diagnostic.

15. The method according to claim 13 or 14, wherein said nanoparticles (6) are composed entirely of one or more non-metallic reducing agents compounded to display electronic band structure.

16. The method according to claim 13 or 14, wherein said nanoparticles (9) are composed by an inner metal core (11) and said outer surface layer (10).

17. The method according to any of claims 13-16, wherein the one or more non-metallic reducing agents comprise one or more oxidic materials selected from the group consisting of: $TiO_2$, $ZrO_2$, $HfO_2$, $V_2O_5$, $Nb_2O_5$, $Ta_2O_5$, $MoO_3$, $WO_3$, $Ga_2O_3$, $In_2O_3$, $SnO_2$, $CeO_2$, $Pr_6O_{11}$, $Nd_2O_3$, $Tb_4O_7$, $Dy_2O_3$, and $Eu_2O_3$.

18. The method according to any of claims 13-16, wherein the non-metallic reducing agents comprise semiconducting materials doped with metal cations selected from the group consisting of: $Ce^{3+}$, $Pr^{3+}$, $Nd^{3+}$, $Eu^{3+}$, or $Yb^{2+}$.

Fig. 1

EP 1 920 784 A1

OH•    H⁺    e⁻

hv

Fig. 2

Fig. 3

9

**European Patent Office**

**PARTIAL EUROPEAN SEARCH REPORT**

which under Rule 45 of the European Patent Convention shall be considered, for the purposes of subsequent proceedings, as the European search report

Application Number

EP 06 12 3943

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/058360 A (PHILIPS INTELLECTUAL PROPERTY [DE]; KONINKL PHILIPS ELECTRONICS NV [NL] 30 June 2005 (2005-06-30) | 1-7,9-12 | INV.<br>A61K41/00 |
| Y | * abstract; figures 1,2,6,7 *<br>* page 4, line 29 - page 5, line 28 *<br>* page 8, line 31 - page 9, line 2 *<br>* claims 1-12; table 2 * | 8 | |
| X | WO 01/91808 A (REGENTS FOR OKLAHOMA STATE UNI [US]) 6 December 2001 (2001-12-06) | 1-5,7,9,10 | |
| Y | * abstract; figure 1 *<br>* page 5, line 9 - page 6, line 23 *<br>* claims 7,8 * | 8 | |
| X | WO 2005/094902 A (PHILIPS INTELLECTUAL PROPERTY [DE]; KONINKL PHILIPS ELECTRONICS NV [NL] 13 October 2005 (2005-10-13)<br>* abstract *<br>* page 2, lines 5-31 *<br>* page 10, line 30 - page 11, line 11; table 1 * | 1-5,7,9,10 | |

-/--

**TECHNICAL FIELDS SEARCHED (IPC)**

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 18 May 2007 | Lahorte, Philippe |

**European Patent
Office**

**PARTIAL EUROPEAN SEARCH REPORT**

Application Number

EP 06 12 3943

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| Y | US 2005/020869 A1 (HAINFELD JAMES F [US] ET AL) 27 January 2005 (2005-01-27)<br>* abstract *<br>* paragraphs [0014] - [0020], [0037], [0038], [0068] - [0073] *<br>----- | 8 | |
| | | | **TECHNICAL FIELDS SEARCHED** (IPC) |

**European Patent Office**

**INCOMPLETE SEARCH SHEET C**

Application Number

EP 06 12 3943

Claim(s) not searched:
        13-18

Reason for the limitation of the search (non-patentable invention(s)):

Article 52 (4) EPC - Method for treatment of the human or animal body by surgery and therapy ("...administering an amount of nanoparticles to at least said tissue...", "subsequently irradiating the animal with ionizing electromagnetic radiation...")

EP 1 920 784 A1

## ANNEX TO THE EUROPEAN SEARCH REPORT
## ON EUROPEAN PATENT APPLICATION NO.

EP 06 12 3943

18-05-2007

| Patent document cited in search report | | Publication date | Patent family member(s) | | Publication date |
|---|---|---|---|---|---|
| WO 2005058360 | A | 30-06-2005 | CN | 1893976 A | 10-01-2007 |
| WO 0191808 | A | 06-12-2001 | AU | 7507801 A | 11-12-2001 |
| | | | EP | 1286705 A2 | 05-03-2003 |
| | | | JP | 2003535063 T | 25-11-2003 |
| WO 2005094902 | A | 13-10-2005 | NONE | | |
| US 2005020869 | A1 | 27-01-2005 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050020869 A

**Non-patent literature cited in the description**

- **WANG et al.** *J. Mater. Chem.,* 2004, vol. 14, 487-493